# EUROPÄISCHE PATENTSCHRIFT

(11) **EP 2 754 209 B1**
(45) Veröffentlichungstag und Bekanntmachung des Hinweises auf die Patenterteilung: **14.09.2016**
(21) Anmeldenummer: 12745655.6
(22) Anmeldetag: 23.07.2012
(51) Int. Cl.: G01N 27/406

(54) **MESSFÜHLER MIT GEHÄUSEDICHTUNG AUS SYNTHESEKAUTSCHUKEN MIT UNTERSCHIEDLICHER ELASTIZITÄT**
SENSOR WITH CHAMBER SEAL MADE OF SYNTHETIC RUBBERS OF DIFFERING ELASTICITY
CAPTEUR POURVU D'UN JOINT DE BOÎTIER EN CAOUTCHOUCS SYNTHÉTIQUES D'ÉLASTICITÉ DIFFÉRENTE

(30) Priorität: 07.09.2011 DE 102011082260
(43) Veröffentlichungstag der Anmeldung: 16.07.2014
(73) Patentinhaber: Robert Bosch GmbH, 70442 Stuttgart (DE)
(72) Erfinder: RATTAY, Bernd, 71254 Ditzingen (DE); SCHNEIDER, Jens, 71229 Leonberg (DE); SOYEZ, Guido, 71636 Ludwigsburg (DE); CLAUSS, Arno, 74348 Lauffen (DE)
(86) Internationale Anmeldenummer: PCT/EP2012/064395
(87) Internationale Veröffentlichungsnummer: WO 2013/034353

(56) Entgegenhaltungen:
- DE-A1- 4 034 072
- DE-A1- 10 121 890
- US-A- 4 141 813

## Beschreibung

### Stand der Technik

Aus dem Stand der Technik ist eine Vielzahl von Messfühlern und Verfahren zur Bestimmung mindestens einer Eigenschaft eines Messgases in einem Messgasraum bekannt. Dabei kann es sich grundsätzlich um beliebige physikalische und/oder chemische Eigenschaften des Gases handeln, wobei eine oder mehrere Eigenschaften erfasst werden können. Die Erfindung wird im Folgenden insbesondere unter Bezugnahme auf eine qualitative und/oder quantitative Erfassung einer Gaskomponente des Gases beschrieben, insbesondere unter Bezugnahme auf eine Erfassung eines Sauerstoffanteils in dem Gas. Der Sauerstoffanteil kann beispielsweise in Form eines Partialdrucks und/oder in Form eines Prozentsatzes erfasst werden. Alternativ oder zusätzlich sind jedoch auch andere Eigenschaften des Gases erfassbar, wie beispielsweise die Temperatur des Gases.

Beispielsweise können derartige Messfühler als so genannte Lambdasonden ausgestaltet sein, wie sie beispielsweise aus Konrad Reif (Hrsg.): Sensoren im Kraftfahrzeug, 1. Auflage 2010, S. 160-165, bekannt sind. Mit Breitband- und Sprunglambdasonden, insbesondere mit planaren Breitband- und Sprunglambdasonden, kann beispielsweise die Sauerstoffkonzentration im Abgas an einem Punkt oder in einem großen Bereich bestimmt und damit auf das Luft-Kraftstoff-Verhältnis im Brennraum geschlossen werden. Alternativ ist jedoch ebenso die Ausbildung als Fingersonde möglich. Die Luftzahl λ beschreibt dieses Luft-Kraftstoff-Verhältnis.

Derartige Messfühler weisen üblicherweise ein Gehäuse, das eine Gehäuseöffnung aufweist, durch die Anschlusskabel herausgeführt sind, und einen in der Gehäuseöffnung angeordneten Dichtkörper auf, der von den Anschlusskabeln durchragt wird. Der Dichtkörper ist ein aus einer homogenen Elastomermasse gefertigter Stopfen, durch den die Anschlusskabel geführt werden und der danach in einem Abschnitt des Gehäuses, der als Metallhülse ausgebildet ist, verstemmt wird. Dies ist beispielsweise in der EP 2 192 403 A1 beschrieben. An derartige Messfühler und insbesondere an den Kabelausgang werden hohe Temperaturanforderungen und Anforderungen an die Wasser- und Gasdichtheit gestellt. Zur Einstellung der elastischen Eigenschaften, die zu einer besseren Verarbeitung und einem besseren Abdichtverhalten führen, werden bei der Herstellung des Dichtkörpers dem Elastomer Weichmacher zugesetzt, die in der Elastomermasse homogen verteilt werden. Weitere Messfühler sind aus der DE 101 21 890 A1, US 4 141 813 A und der DE 40 34 072 A1 bekannt.

Trotz der zahlreichen Vorteile der aus dem Stand der Technik bekannten Messfühler beinhalten diese noch Verbesserungspotenzial. So bewirkt beispielsweise ein hoher Anteil an Weichmachern in der Elastomermasse eine hohe Elastizität, eine gute Verarbeitbarkeit im Neuzustand, ein gutes Abdichtvermögen insbesondere im Bereich der Kabeldurchführung, aber auch einen erhöhten Materialaustrag bei der thermischen Alterung mit einhergehender Schrumpfung und Versprödung. Dadurch können im Kabelausgang des Messfühlers kritische Leckpfade entstehen. Ein niedriger Anteil an Weichmachern in der Elastomermasse hingegen führt zu einer schlechteren Verarbeitbarkeit und einem schlechteren Abdichtvermögen, aber zu einem besseren Alterungsverhalten, da bei thermischer Belastung nur wenig flüchtige organische Komponenten ausgetragen werden und sich die Eigenschaften des Dichtkörpers dementsprechend kaum verändern.

### Offenbarung der Erfindung

Es werden daher ein Messfühler zur Bestimmung mindestens einer Eigenschaft eines Messgases in einem Messgasraum sowie ein Verfahren zu dessen Herstellung vorgeschlagen, welche die Nachteile bekannter Messfühler zumindest weitgehend vermeiden.

Der Messfühler weist ein Gehäuse auf, das eine Gehäuseöffnung aufweist, wobei mindestens ein Anschlusskabel durch die Gehäuseöffnung aus dem Gehäuse herausgeführt ist. Der Messfühler weist weiterhin mindestens einen Dichtkörper auf, insbesondere eine Tülle, wobei der Dichtkörper das Anschlusskabel zumindest teilsweise umschließt. Der Dichtkörper weist mindestens einen ersten Abschnitt und mindestens einen zweiten Abschnitt auf, wobei der erste Abschnitt eine höhere Verformbarkeit aufweist als der zweite Abschnitt.

Die Verformbarkeit kann eine Elastizität und/oder eine Plastizität und/oder eine Kompressibilität umfassen. Der Dichtkörper kann zumindest teilweise in der Gehäuseöffnung angeordnet sein. Der Dichtkörper weist erfindungsgemäß mindestens ein Kunststoffmaterial mit mindestens einem Weichmacher aufweisen, wobei der erste Abschnitt und der zweite Abschnitt einen unterschiedlichen Weichmacheranteil in dem Kunststoffmaterial aufweisen, wobei insbesondere der erste Abschnitt einen höheren Weichmacheranteil aufweist als der zweite Abschnitt. Der zumindest eine Weichmacher kann mit einem Anteil von 0,1 bis 15 % Gewichts-%, bevorzugt 0,25 bis 12,5 Gewichts-% und noch bevorzugter 0,5 bis 10 Gewichts-% in dem Kunststoffmaterial enthalten sein, beispielsweise mit 5 Gewichts-%. Der zumindest eine Weichmacher kann Fluor enthalten. Das Kunststoffmaterial kann mindestens ein Elastomer umfassen. Das Elastomer kann ausgewählt sein aus der Gruppe bestehend aus: Fluorkautschuk, insbesondere Fluorkautschuk mit einem Fluor-Anteil von mindestens 50 Gewichts-%, bevorzugt mindestens 55 Gewichts-% und noch bevorzugter mindestens 60 Gewichts-%, beispielsweise mit 65 Gewichts-%; Perfluorkautschuk, insbesondere Perfluorkautschuk mit einem Fluor-Anteil von mindestens 50 Gewichts-%, bevorzugt mindestens 55 Gewichts-% und noch bevorzugter mindestens 60 Gewichts-%, beispielsweise mit 65 Gewichts-%. Der erste Abschnitt kann das zumindest eine Anschlusskabel umgeben. Der zweite Abschnitt kann koaxial zu dem ersten Abschnitt angeordnet sein. Der zweite Abschnitt kann innerhalb des ersten Abschnitts angeordnet sein. Das Gehäuse kann eine Gehäusewand aufweisen, die die Gehäuseöffnung begrenzt, und der erste Abschnitt kann die Gehäusewand berührt. Das Gehäuse kann eine Längsachse definieren und in einer Schnittebene senkrecht zu der Längsachse gesehen können zwei zweite Abschnitte koaxial zu der Längsachse angeordnet sein, wobei die zwei zweiten Abschnitte durch einen ersten Abschnitt getrennt sind, wobei die Längsachse durch einen zweiten Abschnitt verläuft.

Im Rahmen der vorliegenden Erfindung ist unter der Verformung oder Deformation eines Körpers die Änderung seiner Form infolge der Einwirkung einer äußeren Kraft zu verstehen. Die Verformung kann als Längenänderung, als Winkeländerung, als Flächenänderung oder als Volumenänderung in Erscheinung treten. Die der äußeren Kraft entgegengesetzte Kraft des Körpers ist der Verformungswiderstand. Entsprechend gibt die Verformbarkeit das Maß einer Verformung bei einer bestimmten Krafteinwirkung an. So setzen Körper mit einer höheren Verformbarkeit im Vergleich zu Körpern mit einer niedrigeren Verformbarkeit der äußeren Kraft einen geringeren Verformungswiderstand entgegen, d.h. sie lassen sich mit weniger Kraftaufwand verformen. Die Verformung teilt man in plastische Verformung oder irreversible Verformung und elastische Verformung oder reversible Verformung ein.

Eine irreversible, also dauerhafte, Verformung nennt man plastische Verformung. Die dazu gehörige Eigenschaft eines Werkstoffes nennt man Plastizität bzw. Duktilität. Voraussetzung ist hierbei, dass ein Werkstoff umformbar ist, also eine geringe Sprödigkeit besitzt.

Eine reversible, also eine umkehrbare oder nicht dauerhafte, Verformung nennt man dagegen elastische Verformung. Die dazu gehörige Werkstoffeigenschaft wird Elastizität genannt. Im Rahmen der vorliegenden Erfindung ist daher unter der Elastizität die Eigenschaft eines Körpers oder Werkstoffes zu verstehen, unter Krafteinwirkung seine Form zu verändern und bei Wegfall der einwirkenden Kraft in die Ursprungsform zurückzukehren. Eine höhere Elastizität bedeutet, dass im Vergleich zu einer niedrigeren Elastizität für die gleiche Verformung, wie beispielsweise in Form einer Dehnung oder Kompression, weniger Kraft bzw. für eine stärkere Verformung die gleiche Kraft aufgewendet werden muss. Die Elastizität kann beispielsweise durch den Elastizitätsmodul oder durch den Kompressionsmodul umschrieben werden. Die Kompressibilität ist der Kehrwert des Kompressionsmoduls und beschreibt dieses Verhalten. Je kleiner der Kompressionsmodul desto leichter ist ein Stoff komprimierbar

Im Rahmen der vorliegenden Erfindung sind unter einem Elastomer formfeste, aber elastisch verformbare Kunststoffe zu verstehen. Die elastomeren Kunststoffe können sich bei Zug- und Druckbelastung elastisch verformen, finden aber bei Wegfall der Belastung wieder in ihre ursprüngliche, unverformte Gestalt zurück.

Im Rahmen der vorliegenden Erfindung sind unter Weichmachern Stoffe zu verstehen, die Kunststoffen, Farben und Lacken, Gummi, Klebstoffen und Befilmungsüberzügen zugesetzt werden, um diese weicher, flexibler, geschmeidiger und elastischer im Gebrauch oder der weiteren Verarbeitung zu machen. Weichmacher verschieben den thermoplastischen Bereich eines Kunststoffes hin zu niedrigeren Temperaturen, so dass der Kunststoff also auch im Bereich der Einsatztemperatur die gewünschten "elastischeren" Eigenschaften aufweist.

Der Messfühler kann beispielsweise als Fingersonde ausgeführt werden, also beispielsweise als Lambdasonde mit einem tubusförmigen Aufbau. Da der Messfühler insbesondere im Bereich der Kraftfahrzeugtechnik einsetzbar ist, kann es sich bei dem Messgasraum insbesondere um einen Abgastrakt einer Brennkraftmaschine handeln und bei dem Gas insbesondere um ein Abgas. Die Erfindung ist jedoch ebenso bei jeder Art von Sensorelement oder Messfühler einsetzbar, bei der eine Kabeldurchführung abgedichtet werden muss.

Bei dem erfindungsgemäßen Messfühler liegt keine homogene Verteilung der Materialzusammensetzung des Dichtkörpers vor, sondern beispielsweise ein Gradient der Weichmacherkonzentration. Beispielsweise kann der Dichtkörper aus Fluorkautschuk oder Perfluorkautschuk mit mindestens 60 Gewichts-% Fluor ausgebildet werden. Als Weichmacher kann beispielsweise Dioctylphthalat verwendet werden. Es sind jedoch auch andere niedermolekulare Verbindungen beispielsweise in einem Gewichtsverhältnis von 0,5 bis 5 Gewichts-% in die Polymermasse des Dichtkörpers aus Fluorkautschuk oder Perfluorkautschuk einbringbar, so dass die Elastomereigenschaften beeinflussbar sind. Andere handelsübliche Weichmacher, wie Adipinsäureester oder Sebacinsäureester können je nach Fluorelastomertyp ebenso eingesetzt werden. Insbesondere sind auch fluorhaltige Weichmacher, wie beispielsweise Fluoraromaten, Fluor-Alkane oder Fluor-Polyether, bis zu 10 Gewichts-% in die Basispolymermatrix einbringbar.

So ist es insbesondere vorteilhaft, einen möglichst hohen Anteil an Weichmachern in den äußeren Schichten des Dichtkörpers mit möglichst wenig an Weichmachern im Kern des Dichtkörpers zu kombinieren. Dabei wird bei geringem Gesamtgehalt an Weichmachern eine hohe Elastizität der abdichtenden Randschichten erzielt. Eine hohe Elastizität der Dichtkörperoberfläche ist Voraussetzung für eine gute Abdichtung zwischen den Grenzflächen zwischen Dichtkörper und Gehäuse sowie zwischen Dichtkörper und Anschlusskabel.

### Kurze Beschreibung der Zeichnungen

Weitere optionale Einzelheiten und Merkmale der Erfindung ergeben sich aus der nachfolgenden Beschreibung bevorzugter Ausführungsbeispiele, welche in den Figuren schematisch dargestellt sind. Es zeigen:
- Figur 1: eine Seitenansicht eines Messfühlers im Bereich einer Gehäuseöffnung,
- Figur 2: eine Röntgenaufnahme eines Messfühlers im Bereich einer Gehäuseöffnung,
- Figur 3: eine Querschnittsansicht eines erfindungsgemäßen Messfühlers,
- Figur 4: einen vergrößerten Ausschnitt eines Dichtkörpers im Bereich der Kabeldurchführung,
- Figur 5: eine Modifikation des erfindungsgemäßen Dichtkörpers und
- Figur 6: eine weitere Modifikation des erfindungsgemäßen Dichtkörpers.

### Ausführungsformen der Erfindung

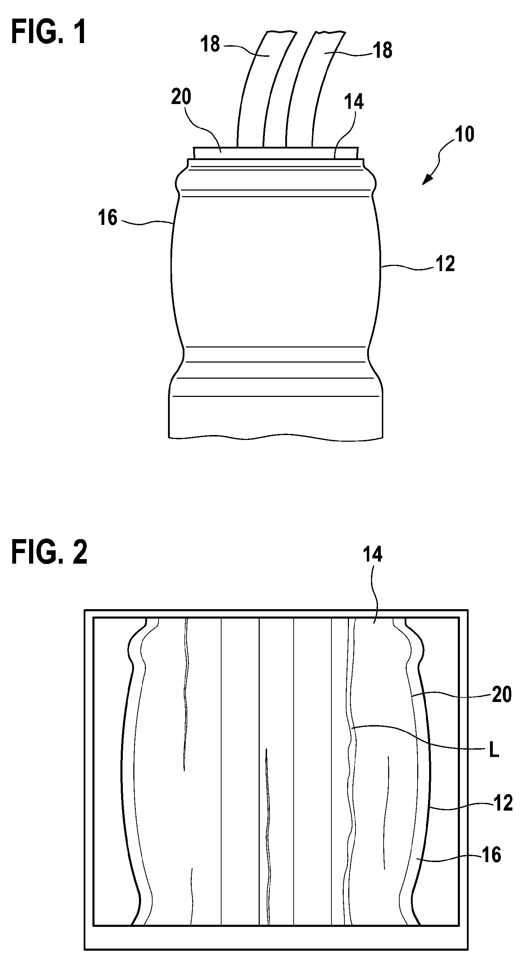

Figur 1 zeigt eine Seitenansicht eines Messfühlers 10. Genauer zeigt Figur 1 einen Teil eines Messfühlers 10. Der Messfühler 10 ist beispielhaft als eine Lambdasonde ausgestaltet. Die Lambdasonde dient zur Steuerung eines Luft-Kraftstoff-Gemisches einer Brennkraftmaschine, um mittels einer Messung der Konzentration des Sauerstoffgehalts im Abgas ein möglichst stöchiometrisches Gemisch einstellen zu können, so dass durch eine möglichst optimale Verbrennung der Schadstoffausstoß minimiert wird. Daher kann es sich bei dem Messgasraum im Rahmen der vorliegenden Erfindung um einen Abgastrakt einer Brennkraftmaschine handeln. Zu diesem Zweck kann der Messfühler 10 in den Abgastrakt hinein ragen. Die Lambdasonde wird nachfolgend als Ausführungsbeispiel für einen Messfühler zur Bestimmung mindestens einer physikalischen und/oder chemischen Eigenschaft eines Messgases, insbesondere der Temperatur oder der Konzentration einer Gaskomponente, insbesondere im Abgas einer Brennkraftmaschine, beschrieben. Insbesondere werden die Unterschiede zu bekannten Messfühlern beschrieben und nicht auf die Betriebsweise eingegangen, da diese beispielsweise aus dem oben genannten Stand der Technik hinreichend bekannt ist und sich die Betriebsweise der Erfindung nicht davon unterscheidet.

Der Messfühler 10 weist ein Gehäuse 12 auf, das eine Gehäuseöffnung 14 aufweist. Die Gehäuseöffnung 14 wird von einer Gehäusewand 16 begrenzt. Durch die Gehäuseöffnung 14 ist zumindest ein elektrisches Anschlusskabel 18 herausgeführt. In der Darstellung der Figur 1 sind beispielsweise zwei von insgesamt vier Anschlusskabeln 18 zu erkennen. In der Gehäuseöffnung 14 ist ein Dichtkörper 20, wie beispielsweise eine Tülle, zumindest teilweise angeordnet. Der Dichtkörper 20 ist vorgesehen, um eine gasdichte und/oder wasserdichte Abdichtung der Gehäuseöffnung 14 zu bilden, so dass Gase und/oder Wasser nicht in das Innere des Gehäuses 12 eindringen können. Der Dichtkörper 20 kann die Gehäusewand 16 berühren, damit auch entlang der Gehäusewand 16 eine gasdichte und/oder wasserdichte Abdichtung gebildet ist. Der Dichtkörper 20 wird von den Anschlusskabeln 18 durchragt. Das Gehäuse 12 ist in dem Bereich des Dichtkörpers 20 zylinderförmig oder hülsenförmig ausgebildet. Insbesondere kann das Gehäuse 12 in dem Bereich des Dichtkörpers 20 aus Metall oder einer Legierung sein. Der Dichtkörper 20 ist in der Gehäuseöffnung 14 mittels einer Verstemmung bzw. Verformung des Gehäuses 12 fixiert.

Würde der Dichtkörper 20 aus einem elastomeren Material, wie beispielsweise Fluorkautschuk oder Perfluorkautschuk, mit einer homogenen Verteilung an Weichmachern ausgebildet sein, so könnte es bei einem zu hohen Anteil an den Weichmachern mit zunehmendem Alter des Dichtkörpers 20 zu Leckpfaden kommen. Dadurch wäre ein Eindringen von Gas oder Wasser in das Innere des Gehäuses 12 zu befürchten. Figur 2 zeigt eine Röntgenaufnahme des Gehäuses 12 im Bereich eines solchen gealterten Dichtkörpers 20, bei dem ein derartiger Leckpfad an der mit L angegebenen Stelle deutlich zu erkennen ist.

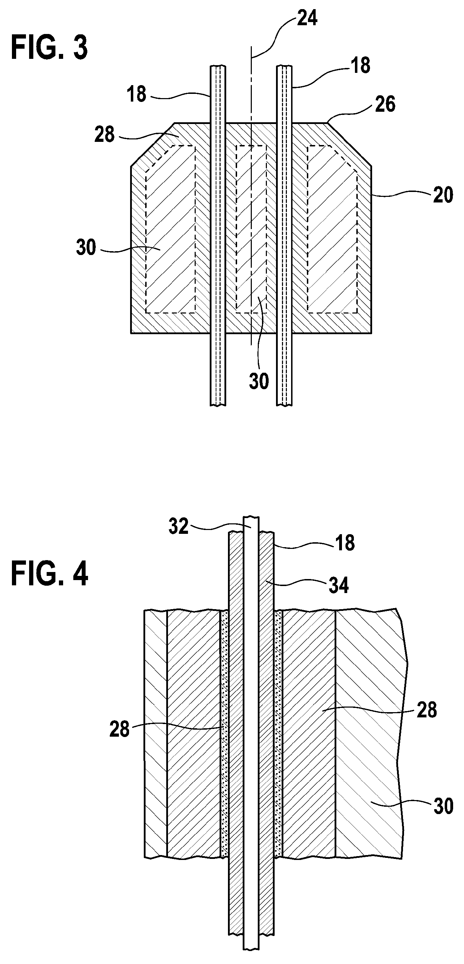

Die vorliegende Erfindung vermeidet derartige Nachteile. Figur 3 zeigt eine beispielhafte Ausführungsform der Erfindung. Insbesondere stellt Figur 3 eine Querschnittsdarstellung durch den Dichtkörper 20 dar. Der Dichtkörper 20 wird von zwei oder mehr Anschlusskabeln 18 durchragt, von denen nur zwei in dieser Darstellung zu sehen sind. Die Anzahl der durchgeführten Anschlusskabel kann variieren. So werden beispielsweise bei Temperaturfühlern oder unbeheizten Lambdasonde üblicherweise zwei Kabel, bei beheizten Sprung- und Breitbandlambdasonden drei, vier oder fünf Kabel und bei speziellen Sonden, wie beispielsweise NOx-Sensoren, sechs und mehr Kabel verwendet. Die Anschlusskabel 18 sind durch Durchgangslöcher 22 in dem Dichtkörper 20 geführt, die parallel zu einer Längsachse 24 des Gehäuses 12 verlaufen. Der Dichtkörper 20 umschließt die Anschlusskabel 18 zumindest teilweise, wobei bei der gezeigten Ausführungsform der Dichtkörper 20 die Anschlusskabel vollständig in Umfangsrichtung, d.h. einer Richtung um die Längsachse, umschließt. Bei der beispielhaften Ausführungsform der vorliegenden Erfindung gemäß Figur 3 weist der Dichtkörper 20 eine im Wesentlichen zylindrische Form auf, wobei der Dichtkörper 20 an einem axialen Ende 26, an dem die Anschlusskabel 18 den Dichtkörper 20 verlassen und das einem Gehäuseinneren abgewandt sein kann, eine Querschnittsverjüngung auf. Der Dichtkörper 20 weist beispielsweise in einer axialen Richtung, d.h. einer Richtung parallel zu der Längsachse 24, eine Abmessung von 9 mm und in einer radialen Richtung, d.h. einer Richtung senkrecht zu der Längsachse 24, eine Abmessung von 12 mm auf. Die fertigungstechnische Toleranz dieser Abmessungen kann 2 mm sein. Es versteht sich, dass die jeweiligen Abmessungen des Dichtkörpers 20 in Abhängigkeit von einer Variation des Abmessungen des Gehäuses 12 variieren können, so dass beispielsweise bei einer Gehäuseöffnung 14 mit einem größeren Durchmesser der Dichtkörper 20 eine entsprechend größere Abmessung in radialer Richtung aufweisen kann.

Insbesondere weist der Dichtkörper 20 einen ersten Abschnitt 28 und zwei zweite Abschnitte 30 auf. Der erste Abschnitt 28 weist im Vergleich zu den zweiten Abschnitten 30 eine höhere Verformbarkeit, wie beispielsweise eine höhere Elastizität, insbesondere Kompressibilität, auf. Wie Figur 3 zeigt, sind in einer Schnittebene senkrecht zu der Längsachse 24 gesehen die zwei zweiten Abschnitte 30 mit niedrigerer Elastizität koaxial zu der Längsachse 24 des Gehäuses 12 angeordnet, wobei die zweiten Abschnitte 30 durch den ersten Abschnitt 28 mit höherer Elastizität getrennt sind. Einer der zwei zweiten Abschnitte 30 ist dabei in der Mitte des Dichtkörpers 20 angeordnet, so dass die Längsachse 24 durch einen zweiten Abschnitt 30 mit niedrigerer Elastizität verläuft. Der zweite Abschnitt 30 mit der niedrigeren Elastizität ist in dem ersten Abschnitt 28 mit der höheren Elastizität aufgenommen bzw. in diesem eingebettet, so dass die Oberfläche des zweiten Abschnitts 30 mit niedrigerer Elastizität vollständig von dem ersten Abschnitt 28 mit höherer Elastizität bedeckt ist. Der erste Abschnitt 28 bildet entsprechend nach außen sowohl in radialer als auch axialer Richtung den Abschluss des Dichtkörpers 20. Die Ausgangskabel 18 verlaufen insbesondere durch den ersten Abschnitt 28 mit höherer Elastizität. Die höhere Elastizität wird dabei dadurch erreicht, dass in dem Material des ersten Abschnitts 28 ein höherer Anteil an Weichmachern enthalten ist als in dem Material des zweiten Abschnitts 30. Insbesondere kann als Weichmacher Dioctylphthalat mit einem Anteil von 0,5 Gewichts-% bis 15 Gewichts-%, bevorzugt 0,25 Gewichts-% bis 12,5 Gewichts-% und noch bevorzugter 0,5 Gewichts-% bis 10 Gewichts-%, in das Material des Dichtkörpers 20 eingebracht werden, beispielsweise mit einem Anteil mit 5 Gewichts-%. Alternativ können als Weichmacher Adipinsäureester oder Sebacinsäureester eingesetzt werden. Ebenso können fluorhaltige Weichmacher, wie beispielsweise Fluoraromaten, Fluor-Alkane oder Fluor-Polyether, mit bis zu 10 Gewichts-% in das Material des Dichtkörpers 20 eingearbeitet werden. Als Material für den Dichtkörper 20 kann dabei ein Kunststoff verwendet werden, wie beispielsweise mindestens ein Elastomer, insbesondere Fluorkautschuk oder Perfluorkautschuk mit einem Fluor-Anteil von mindestens 50 Gewichts-%, bevorzugt mindestens 55 Gewichts-% und noch bevorzugter mindestens 60 Gewichts-%, verwendet werden, beispielsweise mit einem Fluor-Anteil mit 65 Gewichts-%. Derartige Fluorkautschuke weisen beispielsweise 64 bis 74 Gewichts-% Fluor, 21 bis 27 Gewichts-% Ruß und 7 Gewichts-% sonstige, nicht zu deklarierende Bestandteile auf. Solche Fluorkautschuke sind beispielsweise Viton® der DuPont Dow Elastomers, Tecnoflon® der Solvay Plastics, Fluorel® der Dyneon LLC, Daiel® der Daikin America, Inc., erhältlich beispielsweise über die Dätwyler Cables GmbH, Auf der Roos 4-12, 65795 Hattersheim, Deutschland. Solche Perfluorkautschuke sind beispielsweise Kalrez® der DuPont Dow Elastomers, Isolast der Trelleborg Sealing Solutions, Paroflour® der Parker Hannifin GmbH, HPF® der Quarzwerke GmbH, erhältlich beispielsweise über die Dätwyler Cables GmbH, Hattersheim, Deutschland, CTR (Chung Ta Rubber Co., Ltd.), Taiwan oder Doosung Co, Ltd., Korea.

Figur 4 zeigt einen vergrößerten Ausschnitt im Bereich eines Durchgangslochs 22 eines Ausgangskabels 18. Das Ausgangskabel 18 umfasst eine Litze 32 als eigentlichen elektrischen Leiter, die beispielsweise aus Kupfer und/oder Nickel sein kann, und eine Ummantelung 34 aus einem elektrisch isolierenden Material, wie beispielsweise Polytetrafluorethylen. Der Bereich zwischen dem ersten Abschnitt 28 mit höherer Elastizität und der Ummantelung 34 des Ausgangskabels 18 kann mit einer Schicht einer viskosen Paste mit beispielsweise 1 Gewichts-% bis 10 Gewichts-% eines fluorhaltigen Weichmachers gefüllt sein. Mit anderen Worten kann in den verbleibenden Raum eines Durchgangslochs 24 zwischen einer Innenwand desselben und der Ummantelung 34 eine derartige Paste eingebracht sein, wie beispielsweise in Form eines Klebers. Alternativ kann auch ein einschichtiger oder mehrschichtiger Schlauch mit vergleichbaren mechanischen, physikalischen und chemischen Eigenschaften aus einem fluorhaltigen Kunststoff in das Durchgangsloch 24 eingebracht sein. Durch den besonderen erfindungsgemäßen Aufbau eines Dichtkörpers 20 wird erreicht, dass dieser insbesondere im Bereich um die Ausgangskabel 18 hervorragende Dichteigenschaften aufweist, wohingegen aufgrund der zweiten Abschnitte 30 mit niedrigerer Verformbarkeit, insbesondere mit niedrigerer Elastizität, eine auch langzeitstabile Eigenschaft bewirkt wird, da auch bei thermischer Alterung nicht zu viel Materialaustrag durch Verflüchtigung von Weichmachern aus dem Dichtkörper 20 erfolgt und dieser nur geringfügig schrumpft und/oder spröde wird.

Grundsätzlich werden für den Dichtkörper 20 als Basismaterial Fluorelastomere, wie beispielsweise die genannten Fluorkautschuke oder Perfluorkautschuke, verwendet. Diese werden aufgrund ihrer besonderen Temperatur- und Medienresistenzbeständigkeit auch über lange Einsatzzeiten hinweg bevorzugt verwendet, da diese die hohen Temperaturanforderungen an einen Ausgang der Ausgangskabel 18 aus der Gehäuseöffnung 14 von Abgassonden erfüllen können. Beispielsweise können diese eine thermische Belastung bei 300 °C von über 40 Stunden oder bei 250 °C von über 400 Stunden bei einer zuverlässigen Wasser- und Gasdichtheit gewährleisten. Die Ausbildung eines Dichtkörpers 20 aus einem derartigen Fluorelastomer erfolgt dabei in mehren Schritten. Üblicherweise wird ein derartiger Fluorkautschuk als Granulat, Granulatmischung oder als Masse verwendet und mit bekannten Zusatzstoffen aufbereitet, so dass ein zähflüssiges Material entsteht. Grundsätzlich können in das Basismaterial auch weitere feste Füllstoffe, wie beispielsweise Flammruß zur Farbgebung oder basische Oxide, eingebracht werden. Es können auch flüchtige Weichmacher und andere Additive, wie beispielsweise Dioctylphthalat, zur Verbesserung der Verarbeitbarkeit in das Basismaterial eingebracht werden. Dieses zähflüssige Material wird in eine Form gefüllt und durch ein Pressverfahren, dem so genannten Compression Moulding, in die erforderliche Geometrie überführt. Für diese Verfahren eignen sich besonders mittel- bis langkettige Polymere. In Abhängigkeit von der gewünschten Zahl an Ausgangskabeln 18 können dann entsprechende Durchgangslöcher 24 vorgesehen werden, indem dünne Stäbe in der Pressform eingebracht sind. Diese werden nach dem Pressformen wieder aus dem Formkörper herausgezogen, so dass die Durchgangslöcher 24 für die Ausgangskabel 18 vorgesehen sind.

Die unterschiedlichen Elastizitäten der Abschnitte 28 und 30, d.h. der Weichmachergradient bei diesem Beispiel unter Einsatz von Weichmachern, kann grundsätzlich durch eine Nachbehandlung, dem so genannten Curing, erreicht werden. Dabei wird ein thermisch vorgealterter Dichtkörper 20 einer weichmacherhaltigen Lösung oder Gasphase ausgesetzt. Die thermische Voralterung, d.h. das sogenannte Auslagern des fertig geformten Bauteils bei Temperaturen nahe der maximalen Einsatztemperatur, ist ein entscheidender Prozess zur Sicherstellung konstanter Dichtkörpereigenschaften über die Lebensdauer. Dabei kann ein zyklisches Auslagern bevorzugt werden, d.h. ein mehrfaches Aufheizen bis knapp unter die maximale Bauteil-Einsatztemperatur. Dadurch, dass der Dichtkörper 20 der weichmacherhaltigen Lösung oder Gasphase ausgesetzt wird, wird das Lösemittel bzw. werden die Weichmacher in den äußeren Schichten des Dichtkörpers 20 aufgenommen bzw. eingelagert, so dass der gewünschte Weichmachergradient in dem Dichtkörper 20 entsteht. Bei dem Beispiel sind die äußeren Schichten die radial und axial äußeren Oberflächen sowie die Innenwände im Bereich der Durchgangslöcher 24 für die Ausgangskabel 18. Alternativ kann ein noch stärkerer Gradient dadurch erreicht werden, dass der Dichtkörper 20 mit einem weichmacherhaltigen Kleber beschichtet wird. Es ist ebenfalls denkbar, dass der erste Abschnitt 28 und der zweite Abschnitt 30 aus dem selben Kunststoffmaterial gefertigt werden, aber sich in den Elastomergehalten unterscheiden. Insbesondere können die dichtenden Oberflächen des Dichtkörpers 20, d.h. diejenigen Oberflächen, die die Kontaktflächen zu einem anderen Bauteil darstellen, das abgedichtet werden soll, mit einer dünnen Schicht eines weichmacherhaltigen Klebers bedeckt werden. Beispielsweise ist nur eine teilweise Beschichtung von Oberflächen möglich, wie beispielsweise nur der Innenwände der Durchgangslöcher 24 für die Ausgangskabel 18, die besonders kritische Dichtaufgaben übernehmen müssen. Alternativ kann der Dichtkörper 20 auch aus mehreren Bauteilen mit den genannten Eigenschaften zusammengesetzt werden. Nachfolgend werden einige Beispiele für Herstellungsverfahren und Ausführungsformen detaillierter beschrieben, die die genannten Effekte bewirken bzw. die genannten Ausbildungen umsetzen.

Eine erste Variante eines Herstellungsverfahren, um die genannten elastischen Eigenschaften in den jeweiligen Abschnitten des Dichtkörpers 20 zu erzielen, ist beispielsweise, einen einstückigen Dichtkörper 20 aus homogenem Fluorkautschuk oder Perfluorkautschuk in einem ersten Schritt thermisch auszulagern. Vorteilhaft ist eine zyklische Aufheizung, bei der jedoch die maximale Temperatur, die Haltedauer und die Zyklenzahl von dem gewählten Fluorkautschuk oder Perfluorkautschuk abhängen. Beispielsweise werden zehn Zyklen bei 250 °C mit einer Haltedauer von jeweils einer Stunde oder einer Temperatur von 250 °C über mehrere Stunden bevorzugt. Insbesondere kann die thermische Auslagerung in einem evakuierbaren Ofen stattfinden, in dem beispielsweise ein Teil der Zyklen bei Normaldruck unter Normalatmosphäre und ein anderer Teil der Zyklen unter reduziertem Druck durchgeführt wird. In einem zweiten Schritt wird die Oberfläche des ausgelagerten Dichtkörpers 20 in einem Tauchbad aus Lösungsmittel und Weichmachern benetzt. Dabei werden Lösungsmittel und Weichmacher, Lagerzeit und Temperatur so ausgewählt, dass eine Quellung der äußeren Schichten erfolgt, d.h. der Außenflächen und der Innenwände im Bereich der Durchgangslöcher 24, der Kern des Dichtkörpers 20 jedoch unverändert bleibt, da die Weichmacher nicht in tiefer gelegene Schichten des Dichtkörpers 20 eindringen. Je nach Fluorkautschuk oder Perfluorkautschuk können beispielsweise eine Mischung der Alkohole Ethanol und Isopropanol als Lösungsmittel und des Weichmachers Dioctylphthalat bei einer Lagerdauer von 24 Stunden verwendet werden. Dieser Prozessschritt kann beispielsweise in einem Autoklaven bei erhöhtem Druck durchgeführt werden. Alternativ ist eine Oberflächenbehandlung in einer Gasatmosphäre unter Druck möglich. In einem dritten Schritt wird ein derart ausgebildeter Dichtkörper 20 in der Gehäuseöffnung 14 befestigt, wie beispielsweise durch Verstemmen.

Alternativ zu diesen genannten Herstellungsverfahren kann ein einteiliger Dichtkörper 20 aus einem homogenen Fluorkautschuk oder Perfluorkautschuk wie bei der ersten beschriebenen Variante thermisch ausgelagert werden. In einem Folgeschritt werden die äußeren Schichten des Dichtkörpers 20 mit einer viskosen Paste, wie beispielsweise einer Klebstoffzubereitung, eines fluorhaltigen Weichmachers mit beispielsweise 1 Gewichts-% bis 10 Gewichts-% Fluor benetzt. Beispielsweise wird die Klebstoffzubereitung mittels einer Dosiereinrichtung nur partiell auf die Innenwände im Bereich der Durchgangslöcher 24 für die Ausgangskabel 18 aufgebracht, die besonders kritische Abdichtungsbereiche darstellen. Alternativ kann ein einschichtiger oder mehrschichtiger Schlauch mit vergleichbaren mechanischen, physikalischen und chemischen Eigenschaften aus fluorhaltigen Kunststoffen in die Durchgangslöcher 24 eingebracht werden. In einem dritten Schritt wird der so ausgebildete Dichtkörper 20 an der Gehäuseöffnung 14 wie oben beschrieben befestigt.

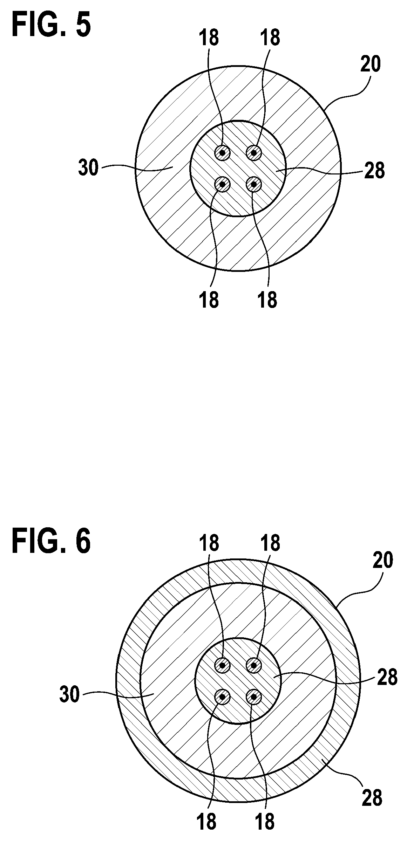

Als weitere Variante ist es möglich, den Dichtkörper 20 als mehrteiliges Bauteil auszubilden. Dabei wird der Gradient an Verformbarkeit, insbesondere Elastizität, bereits bei der Formgebung des Dichtkörpers 20 eingestellt. Dabei wird der erste Abschnitt 28 mit der höheren Verformbarkeit, insbesondere Elastizität, als der Kern, die sogenannte Pille, des Dichtkörpers 20 mit den Durchgangslöchern 24 für die Ausgangskabel 18, an den die höchsten Anforderungen bezüglich Elastizität bzw. Dichtwirkung gestellt werden, aus einem Fluorkautschuk oder Perfluorkautschuk mit einem hohen Weichmacheranteil geformt, in dem bei der Aufbereitung der Kautschuke bereits der Weichmacher oder die Weichmacher zugesetzt werden, d.h. noch vor dem Einfüllen der Kautschukmasse in das Formwerkzeug für das Compression Moulding. In einem zweiten Formgebungsprozess wird der Kern, d.h. der erste Abschnitt 28, mit dem zweiten Abschnitt 30 mit niedrigerer Verformbarkeit, insbesondere Elastizität, in Form eines Rings aus einem härteren, d.h. weniger elastischen, Fluorkautschuk oder Perfluorkautschuk ummantelt. Eine derartige Ausbildung ist beispielsweise in Figur 5 gezeigt, bei der zwei oder mehr Ausgangskabel 18 vorgesehen sind, die durch den ersten Abschnitt 28 mit hoher Elastizität durchgeführt sind, wobei der zweite Abschnitt 30 mit niedriger Elastizität koaxial zu dem ersten Abschnitt 28 mit höherer Elastizität vorgesehen ist. Im Anschluss daran wird der Dichtkörper 20 wie bei der ersten beschriebenen Variante thermisch ausgelagert und dann an bzw. in der Gehäuseöffnung 14 befestigt.

Alternativ ist auch eine dreiteilige Ausbildung eines Dichtkörpers 20 möglich. Figur 6 zeigt eine derartige Ausbildung, bei der eine koaxiale Anordnung der Abschnitte 28 und 30 vorgesehen ist, wobei die ersten Abschnitte 28 mit höherer Elastizität durch einen zweiten Abschnitt 30 mit niedrigerer Elastizität getrennt sind. Ein derartiger mehrteiliger Dichtkörper 20 kann allerdings zu höheren Formgebungskosten, bedingt durch einen zusätzlichen Aufwand beim Pressverfahren, führen.

## Patentansprüche

1. Messfühler (10) mit einem Gehäuse (12), das eine Gehäuseöffnung (14) aufweist, wobei mindestens ein Anschlusskabel (18) durch die Gehäuseöffnung (14) aus dem Gehäuse (12) herausgeführt ist, wobei der Messfühler (10) weiterhin mindestens einen Dichtkörper (20) aufweist, insbesondere eine Tülle, wobei der Dichtkörper (20) das Anschlusskabel (18) zumindest teilweise umschließt, wobei der Dichtkörper (20) mindestens einen ersten Abschnitt (28) und mindestens einen zweiten Abschnitt (30) aufweist, wobei der erste Abschnitt (28) eine höhere Verformbarkeit aufweist als der zweite Abschnitt (30), **dadurch gekennzeichnet, dass** der Dichtkörper (20) mindestens ein Kunststoffmaterial mit mindestens einem Weichmacher aufweist, wobei der erste Abschnitt (28) und der zweite Abschnitt (30) einen unterschiedlichen Weichmacheranteil in dem Kunststoffmaterial aufweisen.

2. Messfühler (10) nach dem vorhergehenden Anspruch, wobei der Dichtkörper (20) zumindest teilweise in der Gehäuseöffnung (14) angeordnet ist.

3. Messfühler (10) nach einem der vorhergehenden Ansprüche, wobei der erste Abschnitt (28) einen höheren Weichmacheranteil aufweist als der zweite Abschnitt (30).

4. Messfühler (10) nach dem vorhergehenden Anspruch, wobei der zumindest eine Weichmacher im ersten Anschnitt (28) mit einem Anteil von 0,1 bis 15 % Gewichts-%, bevorzugt 0,25 bis 12,5 Gewichts-% und noch bevorzugter 0,5 bis 10 Gewichts-% in dem Kunststoffmaterial enthalten ist, beispielsweise mit 5 Gewichts-%.

5. Messfühler (10) nach einem der beiden vorhergehenden Ansprüche, wobei der zumindest eine Weichmacher Fluor enthält.

6. Messfühler (10) nach einem der drei vorhergehenden Ansprüche, wobei das Kunststoffmaterial mindestens ein Elastomer umfasst.

7. Messfühler (10) nach dem vorhergehenden Anspruch, wobei das Elastomer ausgewählt ist aus der Gruppe bestehend aus: Fluorkautschuk, insbesondere Fluorkautschuk mit einem Fluor-Anteil von mindestens 50 Gewichts-%, bevorzugt mindestens 55 Gewichts-% und noch bevorzugter mindestens 60 Gewichts-%, beispielsweise mit 65 Gewichts-%; Perfluorkautschuk, insbesondere Perfluorkautschuk mit einem Fluor-Anteil von mindestens 50 Gewichts-%, bevorzugt mindestens 55 Gewichts-% und noch bevorzugter mindestens 60 Gewichts-%, beispielsweise mit 65 Gewichts-%.

8. Messfühler (10) nach einem der vorhergehenden Ansprüche, wobei der erste Abschnitt (28) das zumindest eine Anschlusskabel (18) umgibt.

9. Messfühler (10) nach einem der vorhergehenden Ansprüche, wobei der zweite Abschnitt (30) koaxial zu dem ersten Abschnitt (28) angeordnet ist.

10. Messfühler (10) nach einem der vorhergehenden Ansprüche, wobei der zweite Abschnitt innerhalb des ersten Abschnitts angeordnet ist.

11. Messfühler (10) nach einem der vorhergehenden Ansprüche, wobei das Gehäuse (12) eine Gehäusewand aufweist, die die Gehäuseöffnung (14) begrenzt, und der erste Abschnitt (28) die Gehäusewand berührt.

12. Messfühler (10) nach einem der vorhergehenden Ansprüche, wobei das Gehäuse (12) eine Längsachse (24) definiert und in einer Schnittebene senkrecht zu der Längsachse (24) gesehen zwei zweite Abschnitte (30) koaxial zu der Längsachse (24) angeordnet sind, wobei die zwei zweiten Abschnitte (30) durch den ersten Abschnitt (28) getrennt sind, wobei die Längsachse (24) durch einen zweiten Abschnitt (30) verläuft.

## Claims

1. Sensor (10) with a housing (12), which has a housing opening (14), at least one connection cable (18) being led out of the housing (12) through the housing opening (14), the sensor (10) also having at least one sealing body (20), in particular a grommet, the sealing body (20) at least partially enclosing the connection cable (18), the sealing body (20) having at least one first portion (28) and at least one second portion (30), the first portion (28) having a greater deformability than the second portion (30), **characterized in that** the sealing body (20) comprises at least one plastics material with at least one plasticizer, the first portion (28) and the second portion (30) having a different proportion of plasticizer in the plastics material.

2. Sensor (10) according to the preceding claim, the sealing body (20) being arranged at least partially in the housing opening (14).

3. Sensor (10) according to one of the preceding claims, the first portion (28) having a higher proportion of plasticizer than the second portion (30).

4. Sensor (10) according to the preceding claim, in the first portion (28) the at least one plasticizer being contained in the plastics material with a proportion of 0.1% to 15% by weight, preferably 0.25 to 12.5% by weight and still more preferably 0.5 to 10% by weight, for example with 5% by weight.

5. Sensor (10) according to one of the two preceding claims, the at least one plasticizer containing fluorine.

6. Sensor (10) according to one of the three preceding claims, the plastics material comprising at least one elastomer.

7. Sensor (10) according to the preceding claim, the elastomer being selected from the group consisting of: fluororubber, in particular fluororubber with a fluorine content of at least 50% by weight, preferably at least 55% by weight and still more preferably at least 60% by weight, for example with 65% by weight; perfluororubber, in particular perfluororubber with a fluorine content of at least 50% by weight, preferably at least 55% by weight and still more preferably at least 60% by weight, for example with 65% by weight.

8. Sensor (10) according to one of the preceding claims, the first portion (28) surrounding the at least one connection cable (18).

9. Sensor (10) according to one of the preceding claims, the second portion (30) being arranged coaxially in relation to the first portion (28).

10. Sensor (10) according to one of the preceding claims, the second portion being arranged within the first portion.

11. Sensor (10) according to one of the preceding claims, the housing (12) having a housing wall which delimits the housing opening (14), and the first portion (28) touching the housing wall.

12. Sensor (10) according to one of the preceding claims, the housing (12) defining a longitudinal axis (24) and two second portions (30) being arranged coaxially in relation to the longitudinal axis (24), as seen in a sectional plane perpendicular to the longitudinal axis (24), the two second portions (30) being separated by the first portion (28), the longitudinal axis (24) extending through one second portion (30).

## Revendications

1. Sonde de mesure (10) présentant un boîtier (12) doté d'une ouverture (14),
au moins un câble de raccordement (18) sortant du boîtier (12) par l'ouverture (14),
la sonde de mesure (10) présentant en outre au moins un corps d'étanchéité (20), en particulier une douille,
le corps d'étanchéité (20) entourant au moins en partie le câble de raccordement (18), le corps d'étanchéité (20) présentant au moins une première partie (28) et au moins une deuxième partie (30),
la première partie (28) présentant une déformabilité plus élevée que la deuxième partie (30), **caractérisée en ce que**
le corps d'étanchéité (20) présente au moins une matière synthétique dotée d'au moins un plastifiant et
**en ce que** la première partie (28) et la deuxième partie (30) présentent des teneurs différentes en plastifiant dans la matière synthétique.

2. Sonde de mesure (10) selon la revendication précédente, dans laquelle le corps d'étanchéité (20) est disposé au moins en partie dans l'ouverture (14) du boîtier.

3. Sonde de mesure (10) selon l'une des revendications précédentes, dans laquelle la première partie (28) présente une teneur en plastifiant plus élevée que la deuxième partie (30).

4. Sonde de mesure (10) selon la revendication précédente, dans laquelle au moins un plastifiant est repris dans la matière synthétique de la première partie (28) à une teneur de 0,1 à 15 % en poids, de préférence de 0,25 à 12,5 % en poids et de façon encore plus préférable de 0,5 à 10 % en poids, et par exemple 5 % en poids.

5. Sonde de mesure (10) selon l'une des deux revendications qui précèdent, dans laquelle le ou les plastifiants contiennent du fluor.

6. Sonde de mesure (10) selon l'une des trois revendications qui précèdent, dans laquelle la matière synthétique comporte au moins un élastomère.

7. Sonde de mesure (10) selon la revendication précédente, dans laquelle l'élastomère est sélectionné dans l'ensemble constitué des caoutchoucs fluorés et en particulier des caoutchoucs fluorés dont la teneur en fluor est d'au moins 50 % en poids, de préférence d'au moins 55 % en poids, de façon encore plus préférable d'au moins 60 % en poids et de préférence de 65 % en poids, les caoutchoucs perfluorés, en particulier les caoutchoucs perfluorés dont la teneur en fluor est d'au moins 50 % en poids, de préférence d'au moins 55 % en poids, de façon encore plus préférable d'au moins 60 % en poids et par exemple de 65 % en poids.

8. Sonde de mesure (10) selon l'une des revendications précédentes, dans laquelle la première partie (28) entoure le ou les câbles de raccordement (18).

9. Sonde de mesure (10) selon l'une des revendications précédentes, dans laquelle la deuxième partie (30) est disposée coaxialement par rapport à la première partie (28).

10. Sonde de mesure (10) selon l'une des revendications précédentes, dans laquelle la deuxième partie est disposée à l'intérieur de la première partie.

11. Sonde de mesure (10) selon l'une des revendications précédentes, dans laquelle le boîtier (12) présente une paroi qui délimite l'ouverture (14) du boîtier, la première partie (28) étant en contact avec la paroi du boîtier.

12. Sonde de mesure (10) selon l'une des revendications précédentes, dans laquelle le boîtier (12) définit un axe longitudinal (24) et dans laquelle deux deuxièmes parties (30) sont disposées coaxialement par rapport à l'axe longitudinal (24) dans un plan de coupe perpendiculaire à l'axe longitudinal (24), les deux deuxièmes parties (30) étant séparées par la première partie (28), l'axe longitudinal (24) s'étendant dans une deuxième partie (30).
